# EUROPEAN PATENT SPECIFICATION

(11) **EP 1 456 375 B1**
(45) Date of publication and mention of the grant of the patent: **01.03.2006**
(21) Application number: 02804303.2
(22) Date of filing: 02.12.2002
(51) Int. Cl.: C12N 15/00, A01K 67/027, A61K 49/00, C12Q 1/68

(54) **SCREENING METHODS EMPLOYING FISH MODEL ASSESSMENT OF VISION**
SCREENING-VERFAHREN UNTER VERWENDUNG EINES FISCH-MODELLS ZUR BEURTEILUNG DER SEHKRAFT
PROCEDES DE TESTS D'EVALUATION DE LA VISION UTILISANT UN MODELE POISSON

(30) Priority: 30.11.2001 GB 0128796
(43) Date of publication of application: 15.09.2004
(73) Proprietor: Daniolabs Limited, Cambridge, Cambridgeshire CB5 9TN (GB)
(72) Inventor: GOLDSMITH, Paul, Cambridge, Cambridgeshire CB2 1TP (GB)
(74) Representative: Walton, Seán Malcolm
(86) International application number: PCT/GB2002/005450
(87) International publication number: WO 2003/048356

(56) References cited:
- WO-A-01/12667
- WO-A-01/51604
- WO-A-01/70944
- WO-A-98/56902
- WO-A-99/42606
- KENNEDY BN, VIHTELIC TS, CHECKLEY L, VAUGHAN KT, HYDE DR.: "Isolation of a zebrafish rod opsin promoter to generate a transgenic zebrafish line expressing enhanced green fluorescent protein in rod photoreceptors." J BIOL CHEM. 2001 APR 27;276(17):14037-43., XP002238860 cited in the application
- BAIER H.: "Zebrafish on the move: towards a behavior-genetic analysis of vertebrate vision." CURR OPIN NEUROBIOL 2000 AUG;10(4):451-5, XP002238861
- LEKVEN AC, HELDE KA, THORPE CJ, ROOKE R, MOON RT.: "Reverse genetics in zebrafish." PHYSIOL GENOMICS 2000 MAR 13;2(2):37-48, XP002238862
- VIHTELIC TS, HYDE DR.: "Zebrafish mutagenesis yields eye morphological mutants with retinal and lens defects." VISION RES 2002 FEB;42(4):535-40, XP001150386
- PETERSON, R.T. ET AL.: "Small molecule developmental screen reveal the logic and timing of vertebrate development " PROCEEDINGS OF THE NATIONAL ACADEMY OF SCIENCES OF USA., vol. 97, no. 24, 21 November 2000 (2000-11-21), pages 12965-12969, XP002238863 NATIONAL ACADEMY OF SCIENCE. WASHINGTON., US ISSN: 0027-8424
- GOLDSMITH P: & HARRIS W.A.: "The zebrafish as a tool for understanding the biology of visual disorders" SEMINAR IN CELL & DEVELOPMENTAL BIOLOGY, XP002238864
- UDVADIA A. & LINNEY E.: "Windows into development : historic , current and future perspectives on transgenic fish" DEV BIOL 2003 APR 1;256(1):1-17, XP002238865
- DEVELOPMENT, vol. 123, 1996, pages 263-273, 1996

## Description

The present invention relates to generation and use of disease models, for instance in assays to identify and investigate genes and substances involved in disease and disease treatment, identification and use of drug targets. In particular, the present invention relates to generation and use of disease models in fish such as zebrafish. The invention further relates to use of fish, e.g. zebrafish, disease models in genetic suppressor screens.

The genes responsible for numerous diseases are now known. In many cases, animal models have been created for these diseases. Most such models are in mammals, such as rats and mice, or in invertebrates such as *Drosophila.* The advantage of using mammalian models is their greater similarity to humans, while the use of invertebrates offers the opportunity to perform sophisticated genetic analysis to identify genes involved in disease.

While the identification of disease causing genes is useful in helping elucidate the mechanisms of disease, it does not necessarily lead to strategies for treatment. It would be very useful to have a model system in which screens can be carried out to identify a gene to cure a previously existing disease. Such a system would be particularly relevant to human disease if performed in a vertebrate and *in vivo.*

The zebrafish is an organism which combines many of the advantages of mammalian and invertebrate model systems. It is a vertebrate and thus more relevant in models of human disease than *Drosophila* or other invertebrates, but unlike other vertebrate models it can be used to perform genetic screens.

The zebrafish has a short maturation period of two to three months and is highly fecund, with a single pair of adults capable of producing 100 to 200 offspring per week. Both embryos and adults are small, cheap and easy to maintain, offering the potential of large scalability. It is possible to introduce random mutations into the zebrafish genome, for example with the use of chemical mutagenesis (Solnica-Krezel et al., Genetics 1994, 136(4): 1401-20). It is also possible to make transgenic fish carrying exogenous genes. Zebrafish expressing a heterologous Ikaros protein has been used to model haematopoiesis and lymphoproliferative disorders (WO 01/40273).

WO99/42606 concerns a method of screening an agent for an angiogenesis activity or cell death activity or toxic activity, comprising administering the agent to a teleost (e.g. zebrafish, medaka, Giant rerio or puffer fish), and detecting a response in the teleost indicating angiogenesis activity or an effect on cell death activity or toxic activity in at least one tissue or organ of the teleost.

WO01/12667 describes use of a transgene to drive marker expression in the eye. The organism may be fish. It suggests making a transgenic animal (which it says may be a fish) by a method comprising introducing a genetic construct for expression of a marker sufficient to visually detect the marker in photoreceptive cells or organ and selecting for transgenesis by visually detecting the marker in a photoreceptive cell or organ.

WO01/51604 (Exelixis) is concerned with providing sensitizer genes such as a tumor gene or an oncogene in a non-human animal (for which zebrafish are mentioned as a passing, hypothetical possibility) in cells where expression is non-lethal. It is proposed to detect changes and compare on mutation or other treatment. The aim is identification of "interactor genes" that, when mutated, specifically kill or reduce the size of target tissue (subject to the sensitizer gene).

Kennedy et al. J Biol Chem (2001) 276, 14037-14043 looks at expression of a marker gene such as GFP in the eye of zebrafish under control of a zebrafish rod opsin promoter.

WO98/56902 discloses use of transgenic fish, including zebrafish, and methods of crossing fish-strains, including strains with mutations, the aim being to identify genes that affect expression of fish genes.

In addition to Zebrafish, other fish such as fugu, goldfish, medaka and giant rerio are amenable to manipulation, mutation and study, and use in aspects and embodiments of the present invention as disclosed herein.

The present invention provides fish (e.g. zebrafish) disease models not only representative of the underlying disease but also amenable for use in a subsequent screen. Advantageously, the present invention allows for study and screening where the disorder or disease would without the invention result in fish not likely to be viable nor able to breed. The invention is generally applicable to any of a variety of diseases and disorders, and a range of examples is specifically set out herein.

The present invention provides means, specifically transgenic fish such as zebrafish, for use in methods of screening for and identifying a gene which, when mutated, alters the activity or effect of a disease gene and thus a disease phenotype. A secondary gene of which mutation affects activity or effect of a primary disease gene is termed a suppressor gene. Suppressor genes represent targets for drugs to treat the disease.

In preferred embodiments of the present invention, a gene, especially a disease gene, is introduced into a fish, e.g. zebrafish, under regulatory control of a promoter that is eye-specific. Thus, genetic modification to introduce a disease gene which would normally lead to animal lethality, lack of viability and/or lack of reproductive ability is effected in such a way that lethality, lack of viability and/or lack of reproductive ability is avoided. For screening purposes it is highly advantageous that the mutated or genetically-modified subject organism is able to reach reproductive age and to generate new offspring also harbouring the genetic alteration.

In other embodiments, a transgenic model fish may be treated with a test substance to screen for a test substance able to affect activity or effect of the transgene, by assessment of vision of the fish, and/or may be subject to mutation to identify a gene in the fish that affects activity or effect of the transgene on vision. Similarly, a model fish may be generated by treatment with a substance, e.g. with a substance that affects vision such as 2,5 hexandedione (arch toxicol 1998: 72: 597-600), N-3-pyridylmethyl-N'-p-nitrophenylurea (Br J Ophthalmol 1988 78:584-90), or a physical treatment, such as light: light induced degeneration has also been described (J Neuobiol 2000 44: 289-307). A model fish provided by means of such a treatment may then be used in a screen for a gene that affects the effect of the treatment and/or for a test substance that affects the effect of the treatment. Effect of a gene or a treatment may be assessed by comparing vision of model and treated and/or mutated fish.

In one aspect, the present invention provides a method of screening for a substance or gene (termed herein "first gene") that affects activity or effect of a second gene, or activity or effect of a treatment, on a fish, the method comprising:
providing, as model fish for screening, (i) fish transgenic for the second gene, wherein the second gene is under regulatory control of an eye-specific promoter and expression of the second gene within the eye of the fish affects vision of the fish, or (ii) fish subject to said treatment, wherein the treatment affects vision of the fish;
mutating said model fish to provide mutated fish or treating said model fish with a test substance to provide treated fish;
comparing vision of mutated fish or treated fish with vision of model fish in order to identify any mutated fish or treated fish with-altered vision compared with model fish;
thereby to identify a test substance that affects activity or effect of the second gene or activity or effect of said treatment, or, by identifying a genetic difference between model fish and mutated fish with such altered vision, to identify a first gene that affects activity or effect of the second gene or activity or effect of said treatment.

The method may comprise mutating model fish transgenic for the second gene to provide mutated fish and identifying a first gene that affects activity or effect of the second gene.

The method may comprise treating with a test substance model fish transgenic for the second gene to provide treated fish and identifying a test substance that affects activity or effect of the second gene.

The method may comprise mutating model fish subject to said treatment to provide mutated fish and identifying a first gene that affects activity or effect of said treatment.

The method-may comprise treating with a test substance model fish subject to said treatment to provide treated fish and identifying a test substance that affects activity or effect of said treatment.

The method may comprise identifying a first gene that lessens activity or effect of the second gene.

The second gene may be a disease gene.

The method may comprise identifying a first gene that enhances or increases activity or effect of the second gene.

The method may comprise identifying a test substance that lessens activity or effect of the second gene or said treatment.

The second gene may be a disease gene.

The method may comprise identifying a test substance that enhances activity or effect of the second gene or said treatment.

Fish may be treated with a substance in a number of ways. Fish may be contacted with a test substance, it may be touched or rubbed on their surface or injected into them. A test substance may be added to water in which they are. A different test substance may be added to each well of a multi-well plate, such as a 96 well plate, to identify that test substance exhibiting a beneficial or deleterious effect. There may be one or multiple fish in each well exposed to the test substance. The test substance may be added prior to the onset of the disease phenotype, concurrent with the onset of the disease phenotype, or subsequent to the onset of the disease phenotype. The same test substance may be added to different wells at different concentration. For example, test substance 1 may be added to well A1 at a concentration of 1mM, to well A2 at a concentration of 100uM, to well A3 at a concentration of 10uM, to well A4 at a concentration of 1uM and to well A5 at a concentration of 0.1uM. Then test substance 2 to well B1 etc. The panel of test substances may be known drugs or new chemical entities.

Additionally, the test substances may be added in combination. For example, well A2 may contain test substance 1 and 2, well A3 test substance 1 and 3, well B2 test substance 2 and 3. Alternatively, every well may contain test substance x, with individual wells containing a panel of additional test substances.

In other options, a population of fish in a petri dish or a-tank may be employed and treated together, e.g. via addition of one or more or a combination of test substances in the water.

A diverse library of drug-like compounds, such as the LOPAC library (Sigma) may be used, or the Chembridge PHARMACOphore diverse combinatorial library. Other targeted libraries against particular targets classes may be used, such as ion channel libraries or G protein libraries. The latter is of particular relevance for vision, given that the phototransduction cascade is the archetypal G protein cascade.

The following drugs are examples that may have some synergistic action together: sodium valproate, ethosuximide, phenytoin, carbamazepine, lamotrigine, gabapentin, phenobarbitone, diazepam, clobazam, tiagabine and levetericatem. Any combination of these and/or other drugs or test substances may be arrayed in every possible combination and tested for synergistic efficacy. Appropriate doses vary from 0.1uM to 10mM, with 100uM being a reasonable starting concentration to assess. This will pick up beneficial effects, with for example, diazepam showing therapeutic effects in monotherapy at 35uM concentration.

According to one aspect of the present invention there is provided a fish, e.g. a zebrafish, fugu, goldfish, medaka or giant rerio transgenic for a disease gene under regulatory control of an eye-specific promoter, wherein expression of the disease gene within the eye of the fish affects vision of the fish.

The promoter used to control eye-specific expression may be inducible, which may facilitate establishment and/or screening of a fish line.

An eye-specific promoter for use in the present invention provides for expression of a disease gene in a component or tissue of the eye involved in one or more of: the passage of a photon of light through the eye, the conversion of photon energy into a biological signal, and the transmission of this signal to the visual centres of the brain. Thus, a promoter for use in the present invention may be functional in one or more of any of the following tissues and cells: conjunctiva, cornea, aqueous humour, vitreous humour, lens, retina, optic nerve, any retinal cell, rod photoreceptor, cone photoreceptor, ganglion cell, glial cell, horizontal cell, bipolar cell and amacrine cell.

Suitable eye-specific promoters are disclosed and discussed elsewhere herein, these being examples, each preferred in an individual embodiment of the invention, while others are available to the skilled person.

In particular embodiments the disease gene when expressed results in a disease phenotype in a dominant fashion. The invention involves placing the gene under the control of an eye-specific promoter, rather than its own natural promoter, to avoid lethality. The disease is then only manifest in the eye, so the fish is blind or at least has impaired vision or visual function compared with wild-type. Visually impaired and blind fish are viable and breed.

An alternative method involves placing the gene under the control of a promoter which is inducible and thus can be switched off and on at will. The disease process can then be switched off before the fish die.

A disease gene to be employed in an embodiment of the present invention may be any gene in wild-type or mutant form which, when expressed in a fish such as zebrafish, in preferred embodiments in a component of the fish eye, results in abnormal development, dysfunction or degeneration of tissue or cell function, thus when expressed in a component of the eye causes abnormal development, dysfunction or degeneration of a tissue or cell of the eye (e.g. as set out already above), this affecting visual function of the fish. In some embodiments, the expression of the disease gene in a component of the eye causes failure of development or degeneration of a part of the visual system. In other embodiments, the expression of the disease gene in a component of the eye causes a component of the visual system to malfunction.

In one preferred embodiment of the present invention, expression of human alpha-synuclein carrying A53T mutation is expressed in fish, e.g. zebrafish, retinal ganglion cells, leading to a degeneration of the ganglion cells. Transmission of visual information to the brain is consequently impaired, and thus visual function of the fish adversely affected. Fish with alpha-synuclein expressed in their retinal ganglion cells perform more poorly than normal on visual testing.

In another preferred embodiment, human rhodopsin carrying P347S mutation is expressed in fish, e.g. zebrafish, photoreceptors, leading to a degeneration of the photoreceptor cells.

Consequently, photons are no longer detected by the retina and thus the vision of the fish is impaired, as determined on visual testing.

In preferred individual embodiments, the dominant acting disease gene is selected from the group consisting of huntingtin, alpha-synuclein, presenilin-1, presenilin-2, TNF, SMN and rhodopsin, either in wild type or mutant forms.

Accession numbers and references for these genes are as follows, and are all incorporated herein by reference:
Huntingtin: accession for wild type human gene OMIM 143100; Huntington's Disease Collaborative Research Group, *Cell* 72: 971-983, 1993. PubMed ID : 8458085, Marsh et al. *Hum Mol Genet* 2000;9(1):13-25;
Alpha-synuclein: accession for wild type human gene XM003494 ; Spillantini et al. *Nature* 1997; 388 (6645) :839-40 ;
Presenilin-1: accession for wild type human gene AH004968 ; Sherrington et al. *Nature* 375 (6534), 754-760 (1995);
Presenilin-2: accession for wild type human gene NM_000447; Levy-Lahad et al. Science 269 (5226), 973-977 (1995), Levy-Lahad et al. Science 269 (5226), 970-973 (1995), Rogaev et al. Nature 376 (6543), 775-778 (1995), Levy-Lahad et al. Genomics 34 (2), 198-204 (1996) ;
TNF: accession for wild type human gene XM_055614 ; Bitsch et al. Glia. 2000 Feb 15;29(4):366-75, Liu et al. Nat Med. 1998 Jan; 4 (1) : 78-83, Probert et al. Proc Natl Acad Sci U S A. 1995 Nov 21;92 (24) : 11294-8;
SOD-1: accession for wild type human gene AY049787; Rosen et al. *Nature* 1993;362 (6415) :59-62, Parkes et al. *Nat Genet* 1998;19(2):171-4;
SMN: accession for wild type human gene XM_041493; Pellizzoni et al. *Cell* 1998, 95(5):615-24, Miguel-Aliaga et al. *FEBS Lett* 2000;486(2):99-102;
Rhodopsin: accession for wild type human gene U49742, Kaushal et al. *Biochemistry* 1994; 33(20):6121-8, Li et al. *Proc Natl Acad Sci U S A* 1996; 93 (24) :14176-81, Colley et al. *Proc Natl Acad Sci U S* A 1995;92(7):3070-4.

The coding sequence of a gene may be placed under the control of (or "operably linked to") the ath5, opsin, rhodopsin or other promoter that expresses in a tissue or cell of the eye. See for example the following references: Mani et al. (2001) J. Biol. Chem. 276(39): 36557-65 (rhodopsin), Knox et al. (1998) FEBS Lett. 423(2): 117-21, Kennedy et al. (2001) J. Biol. Chem. 276 (17) : 14037-43 and Quiambao et al. Vis. Neurosci. (1997) 14 (4) : 617-25 and Kido et al. (1996) Curr. Eye Res. 15(8): 833-44 (opsin) .

The coding sequence of a gene may be placed under the control of an inducible promoter, such as a promoter selected from a heat shock promoter, or a tetracycline or hormone inducible system.

The invention provides for manipulation of nucleic acid in order to modify cells of fish such as zebrafish, as disclosed. Nucleic acid of a disease gene to be expressed in fish in accordance with the invention is to be integrated into the chromosome of cells. Integration may be promoted by inclusion of sequences which promote recombination with the genome, in accordance with techniques available in the art. The disease gene may be heterologous to the fish, e.g. may be heterologous to zebrafish (e.g. mammalian, such as human), and may be in wild-type form or in any allelic or mutant form. The disease gene may be a zebrafish or other fish gene, in wild-type or mutated form, e.g. to provide an extracopy of a zebrafish or other fish gene, such as in a mutated disease form.

Nucleic acid sequences encoding the peptides or polypeptides of the present invention may be readily prepared by the skilled person using the information and references contained herein and techniques known in the art (for example, see Sambrook and Russell "Molecular Cloning, A Laboratory Manual", Third Edition, Cold Spring Harbor Laboratory Press, 2001, and Ausubel et al, Current Protocols in Molecular Biology, John Wiley and Sons, 1992, or later edition thereof). See Detrich et al. (1998) The Zebrafish: Biology. Methods in Cell Biology. Volume 59, and Detrich et al. (1998) The Zebrafish: Genetics and Genomics. Methods in Cell Biology. Volume 60 for techniques of zebrafish maintenance, mutagenesis, transgenesis and mapping.

The desired coding sequence may be incorporated in a construct having one or more control sequences operably linked to the nucleic acid to control its expression. Appropriate regulatory sequences, including promoter sequences, terminator fragments, polyadenylation sequences, enhancer sequences, marker genes and other sequences as appropriate may be included.

Regions responsible for promoter and enhancer activity of a gene known to be expressed in a desirable pattern such as only in the retina, may be isolated by ligating stretches of sequence from upstream of the translation start codon in the gene to a reporter gene. Constructs with deletions in putative promoter and/or enhancer regions are generated and the constructs tested for tissue specific gene expression in transgenic fish, e.g. transgenic zebrafish, fugu, goldfish, medaka or giant rerio.

A selectable marker, for example gene encoding a fluorescent protein such as Green Fluorescent Protein (GFP) may be included to facilitate selection of clones in which the gene construct has inserted into the genome. Where a fluorescent marker is used, embryos may be screened under a fluorescent dissecting microscope. Embryos, or fish into which they grow, may be screened for the presence of a defect resulting from the transgene. In another approach, embryos may be pooled prior to extraction of genomic DNA and analysis of the genomic DNA by PCR and/or restriction enzyme digest. Positive clones may be expanded and developed into breeding fish. These fish may then be bred to produce fish which carry one copy of the gene construct in the germ line. These heterozygous fish may then be bred to produce fish carrying the gene homozygously.

A further aspect provides a method which includes introducing a nucleic acid construct wherein a coding sequence of a desired disease gene is placed under control of an eye-specific promoter into a fish, e.g. zebrafish, embryo cell. DNA may be injected directly in vivo into cells of an early embryo. With the establishment of embryonic stem cell culture, other methods generally referred to without limitation as "transformation", may be employed, for instance selected from any method available in the art, such as-using calcium phosphate transfection, DEAE-Dextran, electroporation, liposome-mediated transfection and transduction using retrovirus or other virus. Marker genes such as antibiotic resistance or sensitivity genes may be used in identifying clones containing nucleic acid of interest, as is well known in the art.

In one particular embodiment, a coding sequence of a disease gene to be introduced into the fish, e.g. zebrafish, is placed under regulatory control of the ath5 promoter. Ath5 is the zebrafish eye-specific ortholog of the Drosophila basic helix-loop-helix transcription factor Atonal and is expressed specifically in retinal ganglion cells (Kay et al. Neuron. 2001 Jun;30(3):725-36). In another embodiment, the promoter of the disease gene is replaced with the opsin promoter (Kennedy et al., (2001) J. Biol. Chem. 276(17): 14037-43) or rhodopsin promoter.

In a further aspect, the present invention provides a method of making a fish, such as a zebrafish, useful in or for use in a screen as disclosed herein and discussed further below. Such a method may comprise providing a gene construct wherein a coding sequence of a disease gene is operably linked to a promoter that is an eye-specific promoter in the fish, e.g. zebrafish, introducing the gene construct into a fish, e.g. zebrafish, embryo, causing or allowing the gene construct to integrate into the fish embryo genome, and growing the fish embryo into a viable fish.

A viable and reproductive fish, e.g. zebrafish, may mate with one or more other fish, establishing a line of fish, e.g. zebrafish, transgenic for the gene construct comprising the disease gene operably linked to, and under regulatory control of, the eye-specific promoter. A line of such fish, e.g. zebrafish, is useful in suppressor gene screens as disclosed.

In order to introduce a disease gene into a fish embryo, e.g a zebrafish embryo, a gene construct is made, using techniques available to those skilled in the art. The construct may be released from a vector by restriction digest, and gel purified, for example by elution in 1xTE (pH8.0) and dilution to a working concentration of 50-100 ug/ml KCl containing a marker dye such as tetramethyl-rhodamine dextran (0.125%). Typically, 1 to 3 nl of this solution may be injected into single celled zebrafish embryos. Several thousand embryos may be injected.

Injected embryos are grown up and then mated with each other or to a non-transgenic wild-type fish. Transmission of the transgene to the subsequent generation is usually mosaic, ranging from 2 to 90%. At least 100 offspring are typically analysed to establish whether the founder fish carriers the transgene.

The injected embryos may be reared and assessed for visual function at intervals (e.g. 2 days) by means of modified optokinetic and optomotor assays, e.g. as discussed further below.

Fish demonstrating visual impairment may be grown up and may be mated with wild-type fish. The parents and offspring may be matched and the offspring similarly assessed for visual dysfunction. Those offspring with visual dysfunction, and hence likely germline transmission of an integrated disease gene construct, can be selectively bred. Some of the offspring may be sacrificed for more detailed analysis, e.g. to confirm the nature of the blindness. This analysis may include *in situ* hybridisation studies using sense and anti-sense probes to the introduced gene to check for expression of the construct in the ganglion cell layer, anatomical assessment such as with plastic sections to check for the degeneration of the ganglion cell layer; and terminal deoxyuridine nucleotide end labelling (TUNEL) to check for apoptotic cell death in the ganglion cell layer.

Families from which fish with the appropriate characteristics came may be maintained through subsequent generations. This maintenance then allows this new mutant strain to be entered into a secondary suppressor screen in accordance with further aspects of the invention.

Another aspect of the present invention provides cells of transgenic fish, such as zebrafish, fugu, goldfish, medaka or giant rerio as disclosed, whether isolated cells or cell lines derived from the fish and optionally immortalised using standard techniques.

A gene such as a disease gene sequence (e.g. heterologous to fish, such as heterologous to zebrafish) to be employed in aspects and embodiments of the present invention may employ a wild-type gene or a mutant, variant or derivative sequence may be employed. The sequence may differ from wild-type by a change which is one or more of addition, insertion, deletion and substitution of one or more nucleotides of the sequence shown. Changes to a nucleotide sequence may result in an amino acid change at the protein level, or not, as determined by the genetic code.

It is well known that pharmaceutical research leading to the identification of a new drug may involve the screening of very large numbers of candidate substances, both before and even after a lead compound has been found. This is one factor which makes pharmaceutical research very expensive and time-consuming. Means for assisting in the screening process can have considerable commercial importance and utility. Such means for screening for substances potentially useful in treating or preventing a disorder or disease is provided.by fish such as zebrafish according to the present invention. Suppressor genes identified using the invention and substances that affect activity of such suppressor genes represent an advance in the fight against disease since they provide basis for design and investigation of therapeutics for in vivo use, as do test substances able to affect activity or effect of a treatment, and substances that affect activity or effect of expression of a disease gene in a fish.

In various further aspects the present invention relates to screening and assay methods and means, and substances identified thereby.

The present inventor has realised that fish such as zebrafish are useful in a secondary suppressor screen. A secondary suppressor screen involves introducing one or more mutations into the genome and screening or selecting for negation or suppression of the effect of a primary mutation.

The principle can be illustrated by way of example, with reference to a hypothetical gene B of which normal function is to control fish size. If this gene is mutated so that it is overactive, bigger fish will ensue. Now take another hypothetical gene S whose normal function is to make cells smaller. If this gene is mutated the cells will be bigger. Thus if a mutation is introduced into the S gene in a cell already harbouring a mutated B gene, the two will cancel each other out and the cell will be normal sized. The mutated S gene suppresses the phenotype of the mutated B gene.

However, there is a problem where gene B is a dominant disease gene, since if this makes the fish non-viable, causes them to die quickly or fail to reproduce, it will not be possible to raise adult fish harbouring a mutation in gene B.

The present invention provides a solution to this problem by restricting expression of the disease gene to the eye, by placing it under the control of an eye-specific promoter. As a result of this restricted expression, the disease process is limited to these specific cells. The fish are viable, can be raised to adulthood and bred, and are thus amenable to use in a secondary suppressor screen.

Furthermore, the invention provides for an accurate, gradable and rapid screening method for the presence of disease activity and degree of disease activity. Degeneration of the retina results in blindness and it is straightforward to assay for blindness and impairment of visual function in fish such as zebrafish in accordance with the present invention.

The fish, e.g. zebrafish, provided by the present invention are useful in screens for suppressor genes that reduce activity or effect of a disease gene. According to a further aspect of the present invention there is provided the use of a fish, e.g. zebrafish, fugu, goldfish, medaka or giant rerio in such a screen.

In a further aspect, the present invention provides a method of screening for a suppressor gene that lessens activity or effect of a disease gene, the method comprising:
providing fish, e.g. zebrafish transgenic for a disease gene under regulatory control of an eye-specific promoter, wherein expression of the disease gene within the eye of the fish affects vision of the fish, as model fish for screening;
subjecting said model fish to mutation to provide mutated fish;
comparing vision of mutated fish with vision of model fish in order to identify any mutated fish with altered vision compared with model fish;
identifying a genetic difference between model fish and any mutated fish with such altered vision, thereby to identify a suppressor gene that lessens activity or effect of the disease gene.

As noted, preferred embodiments of the present invention in its various aspects employ zebrafish.

A fish such as a zebrafish harbouring a disease-causing or phenotype-altering mutation manifested by visual impairment as a result of expression of the disease gene in the eye may be identified on the basis that it does not display the visual impairment or does not display it to the same degree because of the effect of a mutation in a second gene, this mutated second gene suppressing the activity of the disease gene.

Visual function of a fish such as a zebrafish may for example be assayed one of the following ways:
(1) Zebrafish change colour to blend in with background. In a dark environment the fish turn blacker and in a bright environment lighter. Blind fish perceive themselves as being in the dark and thus turn black. This provides a very rapid visual screen - the black fish are blind (Neuhauss).
(2) Zebrafish or other fish optokinetic response can be assayed by passing stripes in front of the eyes. As in human subjects, the resultant reflexive eye movements cannot be suppressed. Thus passage of stripes in front of fish such as zebrafish and the assessment of the presence or absence of optokinetic response provides another assay for visual function. A related visual assay involves the optomotor response. The sophistication of stimuli may be built up to allow a detailed, graded assessment of fish, e.g. zebrafish, visual function (orger). Moreover, the assessment mechanisms allow for the testing of larger numbers of fish in a short period of time.

A moving grating or a movie, e.g. presented as a computer-animated display on a screen, elicits innate optomotor behavior in zebrafish larvae; they swim in the direction of perceived motion (Orger et al. Nat Neurosci 2000 Nov 3(11):1128-33). Zebrafish larvae innately begin responding to moving stimuli shortly after hatching. This is advantageous in a screen of the present invention as disclosed herein, since it allows for rapid determination of the effect of mutation on a model zebrafish. Other fish show similar responses.

Visual function of zebrafish may be determined by considering their colour or background pigmentation, alteration of which is indicative of blindness as discussed. Visual function may be determined by means of observation of response to a pattern of e.g. of stripes passed in front of the eyes of the fish. In a preferred embodiment, black fish expected to be blind and lighter-coloured fish expected to have visual function are segregated on the basis of their colour, prior to determination of the degree or extent of visual function by means of a visual assay.

Assaying for visual function of a fish such as a zebrafish may employ observation of a change in background pigmentation, of visual startle, of optokinetic response and/or optomotor response. The latter two both typically involve passage of a pattern, e.g. horizontal stripes, passed. In an optokinetic assay, fish may be immobilized in 3% methylcellulose and the pattern passed in front of the eyes. In an optomotor assay, fish are free swimming in one or more long, narrow channels, and the pattern is passed along the length of the channels. Fish swim in the perceived direction of motion of the stipes and thus collect at one end of the channel, unless they are blind in which case they are distributed randomly.

A typical powerful stimulus is a 100% contrast square wave filling the entire visual field, and a stripe width of 36 degrees moving at 4 Hz. Limits of vision are identified in Orger et al., supra, although for partially blind fish the limits are less. Degree of visual dysfunction can be determined by decreasing the contrast and/or stripe width of a pattern until the fish no longer respond. Other aspects of visual dysfunction may be analysed by varying the colour of the stripes or other pattern.

The pattern may be stripes or wavy lines moving across the field of vision. Other non-Fourier or second order stimuli may be employed, e.g. as described in Orger et al., *supra.*

Thus, in embodiments of the invention fish such as zebrafish transgenic for a disease gene expressed in the fish under control of an eye-specific promoter may be maintained through several generations. This maintenance allows this strain to be entered into a secondary suppressor screen, through which a secondary mutation is introduced at random into the genome of this strain. The visual function of fish now harbouring both the disease causing mutation and the potentially suppressing secondary mutation may be assessed by means of a modified optomotor or optokinetic assay. Fish in which the visual function is better than the initial subjects of the mutation are candidates for harbouring a second mutation which is suppressing the effect of the primary disease causing mutation. This second mutated gene and its protein product are therapeutic targets for treatment of the disease caused by the primary mutation.

A mutant fish such as a mutant zebrafish transgenic for a disease gene under control of an eye-specific promoter and containing a mutation within a suppressor gene that lessens activity or effect of the disease gene on visual function of the fish is itself useful in a further assay for a test substance able to modulate or affect, preferably potentiate or increase the suppression effect of the suppressor gene. Clearly, the same applies where a mutation in a gene is identified that enhances or increases activity of a second gene.

Of course, the person skilled in the art will design any appropriate control experiments with which to compare results obtained in test assays.

A number of strategies are available to the ordinary skilled person for introducing a secondary mutation into a primary mutant strain. Such strategies include breeding female fish carrying the transgene to homozygosity and then crossing these to F1 or F2 generation males, derived from F0 males exposed to ethylnitrosourea (ENU). The mutagenesis procedure as described by van Eeden et al (Methods Cell Biol 1999 60) typically yields mutation rates of 0.9-3.3x10⁻³ per loci. A starting number of 100 healthy fertile males are considered necessary to obtain 20-30 fertile males after 6 treatments with 3mM ENU.

### Mutagenesis may be performed as follows:

Ethylnitrosourea (ENU) is dissolved in acetic acid to a final concentration of 10mM, as determined by the optical density at 238nm at pH6.0 (extinction coefficient = 5830/M/cm), and then diluted to a working concentration of 3.0mM in 10mM sodium phosphate buffer, pH 6.6. Males which reliably produce fertilised offspring are placed in ENU solution for 1 hour. After the procedure the fish are washed in 2 changes of aquarium water for 1 hour each time, prior to return to the aquarium. The mutagenesis procedure is repeated up to 6 times at weekly intervals.

The frequency of mutations induced is proportional to the exact number of mutagenesis procedures performed. The number of procedures can thus be varied depending on the number of mutations desired per genome.

The actual mutagenesis procedure is best carried out in the dark to minimise the stress to the fish.

Initial progeny from the mutagenised fish are mosaic. The mutagenzed fish are therefore mated 3 times at weekly intervals following the final mutagenesis procedure. Progeny obtained after this will be non-mosaic, since any mutations will have arisen in spermatogonial stem cells.

Other useful mutagenesis agents include gamma- or X-ray-mediated mutagenesis, and retrovirus-mediated insertional mutagenesis.

An alternative method to overcome the problems of lethality and thus allow dominantly acting disease mutations to be entered into a secondary suppressor screen involves placing the mutated gene under the control of an inducible promoter, such as a heat shock promoter, the tetracycline inducible system (Clontech) or a hormonal inducible promoter.

The tetracycline inducible system (Clontech) relies on two different constructs. The tet-on plasmid expresses the tetracycline controlled transactivator, rtTA, consisting of a mutated tet-repressor protein, rTetR, coupled to the VP16 activation domain of the herpes simplex virus (AD). The rtTA protein binds to and activates the tetracycline response element (TRE) part of the second plasmid. The activated TRE acts with the CMV silent promoter to drive expression of the gene of interest. The key to the system is that the rtTA transactivator will only bind the TRE response element in the presence of tetracycline (or its analogue doxycycline). In the absence of tetracycline, binding does not take place, the response element is "off" and protein expression is minimal, such that even toxic proteins can be effectively switched on and off (Harkin et al Cell 1999 97:575-86; Lee et al PNAS 1988 95:11371-6).

The tetracycline inducible system has been used in mammals to provide regulated overexpression of interleukin 11 in the lungs of mice (Ray et al 1997 J Cline Inv 100: 2501-). There was no apparent toxicity to embryos treated with doxycycline in utero, or to pups or adults. In the absence of doxycycline levels of IL-11 were less than 50pg/ml. Doxycycline induction raised levels to greater than 0.3ng/ml. On removal of doxycycline from the drinking water IL-11 levels fell by >80% within 24h. Alterations to the response element are possible, giving even tighter control.

A further modification involves the coupling of the TRE response element to a bi-directional promoter. This allows eGFP to be expressed whenever the target gene is also expressed. This provides an easy visual marker of gene carriage and activation.

Thus in the scenario of a secondary suppressor screen, a mutated gene is introduced into the fish, e.g. zebrafish, under the control of an inducible system (e.g. tetracycline inducible). The gene is switched on by addition of the inducer (e.g. tetracycline) and those fish expressing the gene selected for further study. The gene is then switched off, allowing the fish to be reared through subsequent generations and thus allowing additional mutations to be introduced as part of a secondary suppressor screen.

Accordingly, in a further aspect, the invention provides a method of screening for a suppressor gene that lessens activity or effect of a disease gene, the method comprising:
providing fish such as zebrafish as model fish for screening, which model fish are transgenic for a gene construct wherein a coding sequence a for disease gene that is lethal in fish or renders fish non-viable or non-reproductive is provided under control of an inducible promoter and the model fish are generated and reared under conditions in which the inducible promoter is not induced and/or is repressed;
subjecting the model fish to mutation to provide mutated fish and inducing and/or de-repressing the inducible promoter to cause expression of the disease gene in the model fish;
determining viability and/or reproductivity of mutated fish in which the disease gene is expressed in comparison with model fish in which the disease gene is expressed;
identifying a genetic difference between model fish and any mutated fish with altered viability and/or reproductivity, thereby to identify a suppressor gene that lessens activity or effect of the disease gene.

The suppressed strain of fish will have at least two mutations: the disease-causing/phenotype-inducing mutation resulting from introduction of the disease gene, and the disease-/phenotype-suppressing or repressing mutation.

It should be noted also that in place of identification of a suppressor mutation and gene, the present invention in any of its aspects and embodiments may be used to identify a mutation and gene that enhances or increases the severity of the primary phenotype. This can be used to identify additional genes involved in a particular disease pathway.

Similarly, the present invention may be used to identify a test substance that affects activity or effect of a gene in a fish or a treatment of a fish, e.g. where the gene is a transgene expressed in the eye and the transgene or the treatment has an effect on vision of the fish.

A screening or assay method according to an aspect or embodiment of the present invention may comprise identifying a suppressor gene that lessens activity or effect of a disease gene.

A screening or assay method according to an aspect or embodiment of the present invention may comprise identifying a gene that enhances or increase activity or effect of a second gene, or a test substance that affects activity or effect of the second gene, whether lessening or decreasing, or enhancing or increasing such activity or effect.

Following identification of a gene which affects activity or effect of a second gene, e.g. a suppressor gene, the gene (including a homologue in another species, e.g. human) or encoded gene product may be cloned or otherwise provided in an isolated or purified form,-and may be provided in a composition comprising at least one additional component.

The gene, e.g. suppressor gene, (including a homologue in another species, e.g. human) or a gene product encoded by the gene, e.g. suppressor gene, may be used in a screening system for assaying ability of a test substance to affect activity of the gene or the gene product encoded by the gene.

A test substance that affects activity of a gene, e.g. a suppressor gene, or the gene product encoded by the gene may be provided in a composition comprising at least one additional component.

Following identification of a suppressor gene for a disease gene of interest, or other gene that affects activity or effect of a second gene, the suppressor or other gene and/or an encoded gene product may be employed as a target for identification of potential therapeutics or as a therapeutic in its own right. Also, the nature of the suppressing or other effect may be investigated further.

The suppressor or other gene that affects activity or effect of a second gene may be a novel gene or may be a known gene not previously known to have a function of affecting or suppressing activity or effect of the relevant disease gene. The gene may be one already known or suspected to have function in affecting or suppressing activity, in which case the results from the fish assay add weight to the available evidence. In particular, the fact that the suppression or other effect occurs in vivo increases the confidence for using the gene, or encoded gene product or fragment thereof, or a component in the pathway of action of the gene or gene product, as a drug target. For further investigation and use, a homologue from another species may be used, where available e.g. via use of cloning or screening technology.

The responsible mutation, e.g. suppressive mutation, may be identified by using mapping techniques available in the art, (e.g. see Detrich H.W., Zon L.I. & Westerfield M. (1998) The Zebrafish: Genetics and Genomics. Methods in Cell Biology. Volume 60, pg 182-192).

Thus, to identify the position of the relevant mutation, e.g. a suppressive mutation, the mutant locus is mapped relative to the position of a marker, the position of which is known. DNA markers include short sequences of DNA, cloned genes or other mutations. The current best method in zebrafish involves simple sequence length polymorphisms (SSLPs) as they cover the entire genome at high density. It is therefore possible to map to within 0.5cM, from which either a chromosomal walk may be initiated, further mapping may be undertaken using single strand conformational polymorphisms, or candidate genes selected directly.

### Mapping using SSLP

These markers consist of 2 primers flanking a dinucleotide (CA) repeat. These are extremely variable in length & polymorphic between zebrafish strains. The SSLP mapping involves the following steps:
1. Raising a map cross, identifying mutant carriers, fixing mutant & sibling progeny separately
2. Isolating genomic DNA from both mutants & siblings
3. Genome scanning using pooled DNA from both mutants & siblings to determine linkage group
4. Verifying potential linkages with single embryo DNA
5. Searching for closely linked markers
6. Positioning the mutation on the genetic map by determining the number of recombinations between marker & mutation.

### Isolation of genomic DNA

To extract DNA from single embryos, embryos fixed in 100% methanol are poured into a petri dish. More methanol is added to the dish to ensure the embryos remain covered. Embryos are then pipetted into a 96 well plate: a single embryo per well. A pipette is then used to remove as much methanol as possible from around the embryos. The remaining methanol is then evaporated off on a PCR block set at 70°C for 15 minutes. 25ul of a mix of 250ul proteinase K (17mg/ml, Merck) & 2.25ml 1xTE, is added to each well. The PCR plate is then covered with Hybaid film & heated in a PCR machine for 240 minutes at 55°C, followed by a 10 minute 75°C incubation to inactivate the proteinase K. The plates can be kept at -20°C until needed.

### Genome scanning

Pooled DNA is prepared by taking 10ul from each of 48 single samples, and then diluted to a final concentration of 50ng/ul. Primers for markers-are arranged on a master primer 96 well plate in such a way that the mutant & sibling sample analysed with the same marker will subsequently run adjacent to each other on an agarose gel. The markers selected for the PCR plates are those known to show useful polymorphisms & which evenly span the entire genome.

PCR reactions are then set up in 96 well format. Each well contains 14.28ul PCR mix, 0.16ul each of 20uM forward & reverse primer, 0.4ul of 5U/ul Taq polymerase & 5.0ul of template DNA. PCR is performed with initial denaturing at 94°C for 3 minutes, followed by 35 cycles of denaturing at 94°C for 30 seconds, annealing at 60°C for 30 seconds & primary extension at 72°C for 1 minute. The reaction is completed by a final 5 minute extension at 72°C.

### PCR Mix

0.2mM dATP
0.2mM dCTP
0.2mM dGTP
0.2mM dTTP
in PCR buffer

### PCR buffer (10x)

100mM Tris-HCl, pH 8.3
500mM KCl
15mM MgCl₂
0.1% (w/v) gelatin

### Single-embryo PCR

PCR reactions are set up and performed as above, except that single embryo DNA is used as the template.

The PCR products are assessed for polymorphisms by running out on a 2% agarose gel at 200V for 80 minutes in 1x TBE.

### Mapping using SSCP

This uses single strand DNA. Each strand assumes its thermodynamically preferred conformation. Single nucleotide substitutions may alter the conformation sufficiently for a difference in migration pattern to be detected on a non-denaturing gel. This allows non-SSLP markers tightly linked to the mutation to be analysed.

The protocol used to amplify a marker is as for SSLP mapping. To precipitate the PCR products, 2 volumes of pre-cooled 100% ethanol and 0.1 volume of 3M Na-acetate are added to the PCR product, vortexed well, incubated for at least 20 minutes at - 20°C & centrifuged in a cooled centrifuge at 13000 rpm for 25 minutes. The supernatant is discarded, the DNA pellet air-dried & resuspended in 8ul of ddH₂0. To 5.4ul of PCR product, 0.6ul of denaturing solution & 2.4ul of loading buffer are added & briefly mixed, prior to incubation at 85°C for 10 minutes. The sample is then quickly chilled on ice. 6-8ul of each sample is then loaded onto a native precast acrylamide gel (CleanGel SSCP, ETC Elektrophorese-Technik) & run at 200V & 15°C following the manufacturer's instructions.

### Denaturing solution

10mM EDTA
500mM NaOH

### Loading buffer

2% bromophenol blue
2% xylenecyanol
in formamide

The gels are then stained using a silver staining kit (PlusOne DNA Silver Staining Kit, Pharmacia), as per the manufacturer's instructions.

By these methods the mutation is mapped close enough to select a candidate gene. This gene is then sequenced in both mutant wild-type fish to identify mutations.

If the suppressor or other gene that affects activity or effect of a second gene encodes a protein, it may be that that protein interacts with or binds the second gene, e.g. disease gene, or gene product. Thus, for example, a novel protein-protein binding pair may be identified, immediately presenting the possibility of modulating or affecting such binding as a target for identifying candidate therapeutics.

Where interaction or binding between gene products is to be investigated further or employed in assay methods for identifying further substances able to affect the binding or interaction, suitable approaches are available in the art, for instance techniques involving radioimmunoassay, co-immunoprecipitation, scintillation proximity assay, ELISA methods, and two-hybrid assays (see e.g. Fields and Song, 1989, Nature 340; 245-246), for instance using the two binding domains of the GAL4 transcription factor or the LexA/VP60 system.

Further mutation in the suppressor or other gene may be used to identify variants with enhanced or otherwise altered suppressor function.

Thus, the suppressor gene or other gene, or encoded gene product; in wild-type or a mutated form (which may be a mutated form as identified in the original screen or a further mutated form) may be used in a therapeutic composition.

In various further aspects, the present invention thus provides a pharmaceutical composition, medicament, drug or other composition comprising a suppressor gene or other gene or gene product or substance found to affect the disease gene of interest or suppression of the disease gene of interest, the use of such a material in a method of medical treatment, a method comprising administration of such a material to a patient, e.g. for treatment(which may include preventative treatment) of a medical condition, use of such a material in the manufacture of a composition, medicament or drug for administration for such a purpose, e.g. for treatment of a proliferative disorder, and a method of making a pharmaceutical composition comprising admixing such a material with a pharmaceutically acceptable excipient, vehicle or carrier, and optionally other ingredients.

Whatever the material used in a method of medical treatment of the present invention, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.

Pharmaceutical compositions according to the present invention, and for use in accordance with the present invention, may include, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous or intravenous.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Examples of techniques and protocols mentioned above can be found in Remington's Pharmaceutical Sciences, 16th edition, Osol, A. (ed), 1980.

Vectors such as viral vectors have been used in the prior art to introduce nucleic acid into a wide variety of different target cells. Typically the vectors are exposed to the target cells so that transfection can take place in a sufficient proportion of the cells to provide a useful therapeutic or prophylactic effect from the expression of the desired peptide. The transfected nucleic acid may be permanently incorporated into the genome of each of the targeted cells, providing long lasting effect, or alternatively the treatment may have to be repeated periodically.

A variety of vectors, both viral vectors and plasmid vectors, are known in the art, see US Patent No. 5,252,479 and WO 93/07282. In particular, a number of viruses have been used as gene transfer vectors, including papovaviruses, such as SV40, vaccinia virus, herpesviruses, including HSV and EBV, and retroviruses. Many gene therapy protocols in the prior art have used disabled murine retroviruses.

As an alternative to the use of viral vectors in gene therapy other known methods of introducing nucleic acid into cells includes mechanical techniques such as microinjection, transfer mediated by liposomes and receptor-mediated DNA transfer, also administration of naked DNA or RNA, by simple administration, e.g. injection, of nucleic acid such as a plasmid, for instance to muscle.

Specific disease models which take advantage of such an approach are described. These specific examples are offered by way of illustration and not by way of limitation.

All documents mentioned anywhere in this specification are incorporated by reference.

### EXAMPLE 1 - Parkinson's disease

Parkinson's Disease affects 1 in 100 of the population. 0.5% of multiplex families with Parkinson's Disease harbour a pathogenic mutation in the alpha-synuclein gene (Polymeropolous et al 1996). Whilst it does not appear to be a major risk factor for familial or non-familial Parkinson's Disease it provides a useful general model of Parkinson's Disease pathogenesis. Neuronal overexpression of wildtype asynuclein resulted in progressive accumulation of inclusions in the neocortex, hippocampus and substantia nigra associated with loss of dopaminergic terminals and motor impairment (Masliah et al 2000) and alpha-synuclein is present in the Lewy Bodies of patients with sporadic Parkinson's Disease (Spillantini et al Nature 1997) leading to the conclusion that accumulation of wild type alphasynuclein may be involved in the pathogenesis of Parkinson's Disease. Indeed, the concept of synucleinopathies is emerging as a term to embrace both Parkinson's Disease, Multiple System Atrophy (MSA) and Lewy Body Dementia (LBD). (MSA is the underlying diagnosis in about 10 % of parkinsonian patients; Lewy Body dementia is becoming recognised as the commonest cause of dementia after Alzheimer's Disease).

In an embodiment of the present invention, a mutant alpha-synuclein encoding sequence is ligated to an eye specific promoter and the resulting construct is introduced into the zebrafish genome. In a particular example, mutant human alpha-synuclein is ligated to the zebrafish ath5 promoter. The contruct is introduced into zebrafish embryos and the vision of the resulting fish assayed, to identify fish in which alpha-synuclein is expressed in the eye. These fish are then selected for use in a secondary suppressor screen.

### EXAMPLE 2 - Huntington's Disease

Huntington's Disease is an incurable, dementing condition which affects 4-7/100,000 of population. Genetic testing means affected patients are often identified long before they become symptomatic, but there is still no treatment.

Huntington's disease is due to an expanded CAG repeat sequence in the huntingtin gene with a resultant expanded polyglutamine chain in the protein. A similar mechanism underlies the other triplet repeat diseases described to date (SCA1-8, Kennedy's disease or X-linked bulbo-spinal neuronopathy and DRPLA or dentatorubropallidoluysian atrophy) . The pathogenesis of all the triplet repeat diseases is believed to be similar. Indeed expanded polyglutamine peptides on their own result in cell death and neurodegeneration (Marsh et al HMG 2000 9 13-25). It seems that the remainder of the protein modifies the intrinsic toxicity of the polyglutamine repeat, rendering particular cell groups either more resistant or sensitive (Marsh et al 2000).

In an embodiment of the present invention a zebrafish model of Huntington's Disease amenable for use in a secondary suppressor screen is generated by ligation of mutant human or zebrafish huntingtin to a zebrafish eye specific promoter, such as the ath5 promoter, and subsequent creation of an ath5-promoter-mutant huntingtin zebrafish line. These fish are then useful in a secondary supressor screen to identify genes which alleviate Huntington's Disease.

### EXAMPLE 3 - Spinal muscular atrophy

Spinal muscular atrophy (SMA) is the commonest autosomal recessive disease after cystic fibrosis with an incidence of 1 in 6000 births. It is due to degeneration of the anterior horn cells in the spinal cord with consequent wasting and weakness of all muscles. The rate and severity of muscle wasting varies with milder cases surviving into adulthood. Unfortunately, most commonly death occurs from respiratory failure in early childhood. There are no treatments for SMA available, and none in clinical trial.

Although the pathology is localised to the anterior horn cells, the causative gene, SMN1 or survival of motor neuron, is widely expressed throughout the body. The SMN protein is involved in RNA splicing, partly through its participation in small nuclear ribonucleoprotein (snRNP) assembly in the cytoplasm (Pellizzoni et al Cell 1999 96 1167). The paradox which is challenging SMN researchers is why a protein involved in a process apparently essential to all cells should, in its mutant form, only be deleterious to anterior horn cells.

An embodiment of the present invention comprises creating a zebrafish model of SMA amenable for use in a secondary suppressor screen by ligation of human SMN to a zebrafish eye specific promoter, such as the ath5 promoter, and subsequent generation of an ath5-promoter-mutant SMN zebrafish line. These fish are useful in a secondary supressor screen to identify genes which alleviate SMA. Overexpression of the wild type human SMN in flies is known to result in an SMN phenotype through a dominant negative phenotype (Miguel-Aliaga et al FEBS Letters 2000 486 99-102).

### EXAMPLE 4 - Motor neuron disease

Motor neuron disease (MND) is a devastating neurodegeneration in which progressive dying away of any combination of the motor neurons, anterior horn cells or corticospinal tract results in progressive wasting away of all of the muscles over several years, culminating in a wheelchair-bound patient unable to speak, swallow and eventually breathe. It affects 1 in 1000 of the population. 10% of cases are familial and 20% of these are due,to mutations in the Cu/Zn-superoxide dismutase (SOD-1) gene (Rosen et al 1993). The role of SOD-1 in free radical scavenging suggested that excitotoxic mechanisms could be important in MND pathogenesis. This has been supported by findings in both motor neuron cultures and studies in non-SOD-1 MND patients (e.g. Rothstein et al 1992 and 5; Aoki et al 1998; Rothstein and Kuncl 1995). In drosophila, SOD1 mutations shorten the life span of the fly, whilst overexpression of normal human SOD-1 rescues the mutant phenotype and also extends the life span of wildtype flies, even if the human SOD-1 is only overexpressed in motor neurons (Parkes et al Nat Gen 1998 19 171-4).

A further embodiment of the present provides for creation of a zebrafish model of MND amenable for use in a secondary suppressor screen, involving ligation of mutant human or zebrafish SOD-1 to a zebrafish eye specific promoter, such as the ath5 promoter, and subsequent creation of an ath5-promoter-mutant huntingtin zebrafish line. A secondary suppressor screen may then employ such fish to identify genes which alleviate MND.

### EXAMPLE 5 - Multiple Sclerosis

This chronic disabling condition affects 1 in 100 of the population. Current immunomodulatory therapies such as betaIFN have only weak efficacy in a subset of sufferers. Moreover, they need to be given subcutaneously and frequently have side-effects.

Overexpression of TNF alpha has been validated as an experimental model system in mice (Probert et al. (1995) Proc. Natl. Acad. Sci. USA 92 (24) : 11294-8). A zebrafish model of MS amenable for use in a secondary suppressor screen may be generated as an embodiment of the present invention by ligation of a TNFalpha gene to a zebrafish eye specific promoter, such as the ath5 promoter, and subsequent creation of an ath5-promoter- TNFalpha zebrafish line. Putting these fish into a secondary supressor screen allows for identifying genes which alleviate MS. This model is particularly useful because the ganglion cells are unique in the retina, in that their axons give rise to the optic nerve, a common site of disease in MS. Such a model would not function if the TNFalpha gene was overexpressed in any other retinal cell type. It is the peculiar property of the ganglion cells giving rise to a central nervous system tract of myelinated tissue which makes this possible. The onset of disease in zebrafish would therefore result in demyelination of the optic nerve, hence blindness and an easily scoreable MS phenotype.

## Claims

1. A method of screening for a substance or gene (termed herein "first gene") that affects activity or effect of a second gene, on a fish, the method comprising:
providing, as model fish for screening, fish transgenic for the second gene, wherein the second gene is under regulatory control of an eye-specific promoter and expression of the second gene within the eye of the fish affects vision of the fish;
mutating said model fish to provide mutated fish or treating said model fish with a test substance to provide treated fish;
comparing vision of mutated fish or treated fish with vision of model fish in order to identify any mutated fish or treated fish with altered vision compared with model fish;
thereby to identify a test substance that affects activity or effect of the second gene, or, by identifying a genetic difference between model fish and mutated fish with such altered vision, to identify a first gene that affects activity or effect of the second gene or activity.

2. A method according to claim 1 comprising mutating model fish transgenic for the second gene to provide mutated fish and identifying a first gene that affects activity or effect of the second gene.

3. A method according to claim 1 comprising treating with a test substance model fish transgenic for the second gene to provide treated fish and identifying a test substance that affects activity or effect of the second gene.

4. A method according to claim 1 comprising identifying a first gene that lessens activity or effect of the second gene.

5. A method according to claim 4 wherein the second gene is a disease gene.

6. A method according to claim 1 comprising identifying a first gene that enhances or increases activity or effect of the second gene.

7. A method according to claim 1 comprising identifying a test substance that lessens activity or effect of the second gene

8. A method according to claim 7 wherein the second gene is a disease gene.

9. A method according to claim 1 comprising identifying a test substance that enhances activity or effect of the second gene.

10. A method according to claim 1 wherein the eye-specific promoter is inducible.

11. A method according to claim 10 wherein the eye-specific promoter is selected from the group consisting of ath5, opsin, rhodopsin, nocturnin, PAX6 or Brn3 promoter, or a specific phototransduction cascade promoter.

12. A method according to any one of claims 1 to 11 wherein the second gene is a disease gene and is selected from the group consisting of human SMN, a truncated form of SMN, wild type or mutant huntingtin, wild type or mutant SOD-1, TNF alpha, wildtype or mutant alpha synuclein, wild type or mutant rhodopsin, wild type or mutant ELOVL4 or wild type or mutant presenilin-1 or -2.

13. A method according to claim 1 comprising identifying a first gene that is a suppressor gene of a second gene and in addition to performing the steps set out in claim 1 providing the suppressor gene or a gene product encoded by the suppressor gene in a composition comprising at least one additional component.

14. A method according to claim 13 further comprising in addition to performing the steps set out in claim 13 providing the suppressor gene or a gene product encoded by the suppressor gene in a screening system for assaying ability of a test substance to affect activity of the suppressor gene or the gene product encoded by the suppressor gene.

15. A method according to claim 14 further comprising in addition to the performing the steps set out in claim 13 and claim 14 providing a test substance that affects activity of the suppressor gene or the gene product encoded by the suppressor gene in a composition comprising at least one additional component.

16. A method according to claim 1 comprising identifying a test substance that affects activity or effect of a second gene, and in addition to performing the steps set out in claim 1 providing the test substance in a composition comprising at least one additional component.

17. A method according to any one of claims 1 to 16 wherein the fish is selected from the group consisting of zebrafish, fugu and goldfish.

18. A method according to claim 17 wherein the fish is zebrafish.

19. A fish transgenic for a disease gene under regulatory control of an eye-specific promoter, wherein expression of the disease gene within the eye of the fish affects vision of the fish.

20. A fish according to claim 19 wherein the eye-specific promoter is inducible.

21. A fish according to claim 20 wherein the eye-specific promoter is selected from the group consisting of ath5, opsin, rhodopsin, nocturnin, PAX6 or Brn3 promoter, or a specific phototransduction cascade promoter.

22. A fish according to any one of claims 19 to 21 wherein the disease gene is selected from the group consisting of human SMN, a truncated form of SMN, wild type or mutant huntingtin, wild type or mutant SOD-1, TNF alpha, wildtype or mutant alpha synuclein, wild type or mutant rhodopsin, wild type or mutant ELOVL4 or wild type or mutant presenilin-1 or -2.

23. A fish according to any one of claims 19 to 22 wherein the fish is selected from the group consisting of zebrafish, fugu and goldfish.

24. A fish according to claim 23 wherein the fish is a zebrafish.

25. Use of a fish according to any one of claims 19 to 24 in a screen for identifying a first gene that affects activity or effect of a second gene.

26. Use according to claim 25 wherein the first gene is a suppressor gene of activity or effect of the second gene, wherein the second gene is a disease gene.

27. Use of a fish according to any one of claims 19 to 24 in a screen for identifying a test substance that affects activity or effect of a second gene.

## Patentansprüche

1. Verfahren zum Screenen auf eine Substanz oder ein Gen (hierin als "erstes Gen" bezeichnet), die/das die Aktivität oder Wirkung eines zweiten Gens an einem Fisch beeinflusst, umfassend:
das Bereitstellen, als Modellfisch zum Screenen, eines Fisches, der für das zweite Gen transgen ist, worin das zweite Gen unter Regulationskontrolle eines Augen-spezifischen Promotors steht und Expression des zweiten Gens innerhalb des Auges des Fisches das Sehvermögen des Fisches beeinflusst;
das Mutieren des Modellfisches, um einen mutierten Fisch bereitzustellen, oder das Behandeln des Modellfisches mit einer Testsubstanz, um einen behandelten Fisch bereitzustellen;
das Vergleichen des Sehvermögens des mutierten Fisches oder behandelten Fisches mit dem Sehvermögen eines Modellfisches, um im Vergleich mit dem Modellfisch jeglichen mutierten Fisch oder behandelten Fisch mit verändertem Sehvermögen zu identifizieren;
um hierdurch eine Testsubstanz zu identifizieren, die die Aktivität oder Wirkung des zweiten Gens beeinflusst, oder um, durch Identifikation eines genetischen Unterschieds zwischen Modellfisch und mutiertem Fisch mit solch einem veränderten Sehvermögen, ein erstes Gen zu identifizieren, das die Aktivität oder Wirkung des zweiten Gens oder die Aktivität beeinflusst.

2. Verfahren nach Anspruch 1, umfassend die Mutation eines Modellfisches, der für das zweite Gen transgen ist, um einen mutierten Fisch bereitzustellen, und die Identifikation eines ersten Gens, das die Aktivität oder Wirkung des zweiten Gens beeinflusst.

3. Verfahren nach Anspruch 1, umfassend die Behandlung des Modellfisches, der für das zweite Gen transgen ist, mit einer Testsubstanz, um einen behandelten Fisch bereitzustellen, und die Identifikation einer Testsubstanz, die die Aktivität oder Wirkung des zweiten Gens beeinflusst.

4. Verfahren nach Anspruch 1, umfassend die Identifikation eines ersten Gens, das die Aktivität oder Wirkung des zweiten Gens reduziert.

5. Verfahren nach Anspruch 4, worin das zweite Gen ein Krankheitsgen ist.

6. Verfahren nach Anspruch 1, umfassend die Identifikation eines ersten Gens, das die Aktivität oder Wirkung des zweiten Gens fördert oder steigert.

7. Verfahren nach Anspruch 1, umfassend die Identifikation einer Testsubstanz, die die Aktivität oder Wirkung des zweiten Gens reduziert.

8. Verfahren nach Anspruch 7, worin das zweite Gen ein Krankheitsgen ist.

9. Verfahren nach Anspruch 1, umfassend die Identifikation einer Testsubstanz, die die Aktivität oder Wirkung des zweiten Gens steigert.

10. Verfahren nach Anspruch 1, worin der Augen-spezifische Promotor induzierbar ist.

11. Verfahren nach Anspruch 10, worin der Augen-spezifische Promotor aus der aus ath5-, Opsin-, Rhodopsin-, Nocturnin-, PAX6- oder Brn3-Promotor bestehenden Gruppe ausgewählt ist oder ein spezifischer Phototransduktions-Kaskadenpromotor ist.

12. Verfahren nach einem der Ansprüche 1 bis 11, worin das zweite Gen ein Krankheitsgen ist und aus der aus menschlichem SMN, einer trunkierten Form von SMN, Wildtyp- oder mutiertem Huntingtin, Wildtyp- oder mutiertem SOD-1, TNF-alpha, Wildtyp- oder mutiertem alpha-Synuclein, Wildtyp- oder mutiertem Rhodopsin, Wildtyp- oder mutiertem ELOVL4 oder Wildtyp- oder mutiertem Presenilin-1 oder -2 bestehenden Gruppe ausgewählt ist.

13. Verfahren nach Anspruch 1, umfassend die Identifikation eines ersten Gens, das ein Suppressorgen eines zweiten Gens ist, und zusätzlich zur Durchführung der in Anspruch 1 genannten Schritte das Bereitstellen des Suppressorgens oder eines Genprodukts, für das das Suppressorgen kodiert, in einer Zusammensetzung, die zumindest eine zusätzliche Komponente umfasst.

14. Verfahren nach Anspruch 13, zusätzlich zur Durchführung der in Anspruch 13 genannten Schritte weiters umfassend die Bereitstellung des Suppressorgens oder eines Genprodukts, für das das Suppressorgen kodiert, in einem Screening-System zum Testen der Fähigkeit einer Testsubstanz, die Aktivität des Suppressorgens oder des Genprodukts, für das das Suppressorgen kodiert, zu beeinflussen:

15. Verfahren nach Anspruch 14, zusätzlich zur Durchführung der in Anspruch 13 und Anspruch 14 genannten Schritte weiters umfassend die Bereitstellung einer Testsubstanz, die die Aktivität des Suppressorgens oder des Genprodukts, für das das Suppressorgen kodiert, beeinflusst, in einer Zusammensetzung, die zumindest eine zusätzliche Komponente umfasst.

16. Verfahren nach Anspruch 1, umfassend die Identifikation einer Testsubstanz, die die Aktivität oder Wirkung eines zweiten Gens beeinflusst, und zusätzlich zur Durchführung der in Anspruch 1 genannten Schritte die Bereitstellung der Testsubstanz in einer Zusammensetzung, die zumindest eine zusätzliche Komponente umfasst.

17. Verfahren nach einem der Ansprüche 1 bis 16, worin der Fisch aus der aus Zebrafisch, Fugu und Goldfisch bestehenden Gruppe ausgewählt ist.

18. Verfahren nach Anspruch 17, worin der Fisch ein Zebrafisch ist.

19. Fisch, der für ein Krankheitsgen transgen ist, unter der Regulationskontrolle eines Augen-spezifischen Promotors, worin die Expression des Krankheitsgens innerhalb des Auges des Fisches das Sehvermögen des Fisches beeinflusst.

20. Fisch nach Anspruch 19, worin der Augen-spezifische Promotor induzierbar ist.

21. Fisch nach Anspruch 20, worin der Augen-spezifische Promotor aus der aus ath5-, Opsin-, Rhodopsin-, Nocturnin-, PAX6- oder Brn3-Promotor bestehenden Gruppe ausgewählt ist oder ein spezifischer Phototransduktions-Kaskadenpromotor ist.

22. Fisch nach einem der Ansprüche 19 bis 21, worin das Krankheitsgen aus der aus menschlichem SMN, einer trunkierten Form von SMN, Wildtyp- oder mutiertem Huntingtin, Wildtyp- oder mutiertem SOD-1, TNF-alpha; Wildtyp- oder mutiertem alpha-Synuclein, Wildtyp- oder mutiertem Rhodopsin, Wildtyp- oder mutiertem ELOVL4 oder Wildtyp- oder mutiertem Presenilin-1 oder -2 bestehenden Gruppe ausgewählt ist.

23. Fisch nach einem der Ansprüche 19 bis 22, worin der Fisch aus der aus Zebrafisch, Fugu und Goldfisch bestehenden Gruppe ausgewählt ist.

24. Fisch nach Anspruch 23, worin der Fisch ein Zebrafisch ist.

25. Verwendung eines Fisches nach einem der Ansprüche 19 bis 24 in einem Screen zur Identifikation eines ersten Gens, das die Aktivität oder Wirkung eines zweiten Gens beeinflusst.

26. Verwendung nach Anspruch 25, worin das erste Gen ein Suppressorgen der Aktivität oder Wirkung des zweiten Gens ist, worin das zweite Gen ein Krankheitsgen ist.

27. Verwendung eines Fisches nach einem der Ansprüche 19 bis 24 in einem Screen zur Identifikation einer Testsubstanz, die die Aktivität oder Wirkung eines zweiten Gens beeinflusst.

## Revendications

1. Méthode de test d'évaluation pour une substance ou un gène (que l'on appelle ici "premier gène") qui affecte l'activité ou l'effet d'un second gène, sur un poisson, la méthode consistant à:
prévoir, en tant que poisson modèle pour le test d'évaluation, un poisson transgénique pour le second gène, où le second gène est sous contrôle de régulation d'un promoteur spécifique de l'oeil et l'expression du second gène dans l'oeil du poisson affecte la vision du poisson;
muter ledit poisson modèle pour produire un poisson muté ou traiter ledit poisson modèle avec une substance de test pour produire un poisson traité;
comparer la vision du poisson muté ou du poisson traité à la vision du poisson modèle afin d'identifier tout poisson muté ou poisson traité ayant une vision altérée en comparaison avec le poisson modèle;
pour ainsi identifier une substance de test qui affecte l'activité ou l'effet du second gène ou, en identifiant une différence génétique entre le poisson modèle et le poisson muté ayant cette vision altérée, pour identifier un premier gène qui affecte l'activité ou l'effet du second gène sur l'activité.

2. Méthode selon la revendication 1 comprenant la mutation d'un poisson modèle transgénique pour le second gène pour produire un poisson muté et l'identification d'un premier gène qui affecte l'activité ou l'effet du second gène.

3. Méthode selon la revendication 1 consistant à traiter avec une substance de test un poisson modèle transgénique pour le second gène pour produire un poisson traité et à identifier une substance de test qui affecte l'activité ou l'effet du second gène.

4. Méthode selon la revendication 1 comprenant l'identification d'un premier gène qui diminue l'activité ou l'effet du second gène.

5. Méthode selon la revendication 4 où le second gène est un gène de maladie.

6. Méthode selon la revendication 1, comprenant l'identification d'un premier gène qui améliore ou augmente l'activité ou l'effet du second gène.

7. Méthode selon la revendication 1, comprenant l'identification d'une substance de test qui diminue l'activité ou l'effet du second gène.

8. Méthode selon la revendication 7, où le second gène est un gène de maladie.

9. Méthode selon la revendication 1, comprenant l'identification d'une substance de test qui améliore l'activité ou l'effet du second gène.

10. Méthode selon la revendication 1 où le promoteur spécifique de l'oeil est inductible.

11. Méthode selon la revendication 10 où le promoteur spécifique de l'oeil est sélectionné dans le groupe consistant en ath5, opsine, rhodopsine, nocturnine, PAX6 ou Brn3 comme promoteur, ou un promoteur d'une cascade de phototransduction spécifique.

12. Méthode selon l'une quelconque des revendications 1 à 11 où le second gène est un gène de maladie et est sélectionné dans le groupe consistant en SMN humain, une forme tronquée de SMN, huntingtine du type sauvage ou mutant SOD -1 du type sauvage ou mutant, TNF alpha, alpha synucléine du type sauvage ou mutant, rhodopsine du type sauvage ou mutant , ELOVL4 du type sauvage ou mutant ou préséniline-1 ou -2 du type sauvage ou mutant.

13. Méthode selon la revendication 1, consistant à identifier un premier gène qui est un gène suppresseur d'un second gène et de plus à accomplir les étapes indiquées à la revendication 1 fournissant le gène suppresseur ou un produit du gène codé par le gène suppresseur dans une composition comprenant au moins un composant additionnel.

14. Méthode selon la revendication 13 consistant de plus à accomplir les étapes indiquées à la revendication 13 fournissant le gène suppresseur ou un produit du gène codé par le gène suppresseur dans un système de test d'évaluation pour évaluer l'aptitude d'une substance de test à affecter l'activité du gène suppresseur ou du produit du gène codé par le gène suppresseur.

15. Méthode selon la revendication 14 consistant de plus, en plus de l'accomplissement des étapes indiquées à la revendication 13 et à la revendication 14 à fournir une substance de test qui affecte l'activité du gène suppresseur ou du produit du gène codé par le gène suppresseur dans une composition comprenant au moins un composant additionnel.

16. Méthode selon la revendication 1 comprenant l'identification d'une substance de test qui affecte l'activité ou l'effet d'un second gène et de plus à accomplir les étapes indiquées à la revendication 1 procurant la substance de test à une composition comprenant au moins un composant additionnel.

17. Méthode selon l'une quelconque des revendications 1 à 16, où le poisson est sélectionné dans le groupe consistant en poisson zèbre, fugu et poisson rouge.

18. Méthode selon la revendication 17 où le poisson est un poisson zèbre.

19. Poisson transgénique pour un gène de maladie sous le contrôle régulateur d'un promoteur spécifique de l'oeil où l'expression du gène de maladie dans l'oeil du poisson affecte la vision du poisson.

20. Poisson selon la revendication 19, où le promoteur spécifique de l'oeil est inductible.

21. Poisson selon la revendication 20, où le promoteur spécifique de l'oeil est sélectionné dans le groupe consistant en ath5, opsine, rhodopsine, nocturnine, PAX6 ou Brn3 comme promoteur, ou bien un promoteur de cascade de phototransduction spécifique.

22. Poisson selon l'une quelconque des revendications 19 à 21 où le gène de maladie est sélectionné dans le groupe consistant en SMN humain, forme tronquée de SMN, huntingtine du type sauvage ou mutant , SOD -1 du type sauvage ou mutant, TNF alpha, alpha synuc léine du type sauvage ou mutant , rhodopsine du type sauvage ou mutant , ELOVL4 du type sauvage ou mutant ou préséniline-1 ou -2 du type sauvage ou mutant.

23. Poisson selon l'une quelconque des revendications 19 à 22 où le poisson est sélectionné dans le groupe consistant en poisson zèbre, fugu et poisson rouge.

24. Poisson selon la revendication 23 où le poisson est un poisson zèbre.

25. Utilisation d'un poisson selon l'une quelconque des revendications 19 à 24 dans un test d'évaluation pour identifier un premier gène qui affecte l'activité ou l'effet d'un second gène.

26. Utilisation selon la revendication 25 où le premier gène est un gène suppresseur de l'activité ou de l'effet du second gène, où le second gène est un gène de maladie.

27. Utilisation d'un poisson selon l'une quelconque des revendications 19 à 24 dans un test d'identification pour identifier une substance de test qui affecte l'activité ou l'effet d'un second gène.
